# EUROPÄISCHE PATENTANMELDUNG

(11) **EP 3 199 168 A1**
(43) Veröffentlichungstag der Anmeldung: **02.08.2017**
(21) Anmeldenummer: 17153500.8
(22) Anmeldetag: 27.01.2017
(51) Int. Cl.: A61K 33/26, A61K 31/295, A61K 31/375, A61K 31/519, A61K 47/55, A61K 9/16, A61P 3/02

(54) **ARZNEIMITTEL ZUR BEHANDLUNG VON EISENMANGELZUSTÄNDEN MIT FOLSÄUREDEFIZIT**

(30) Priorität: 28.01.2016 EP 16153151
(71) Anmelder: G.L. Pharma GmbH, 8502 Lannach (AT)
(72) Erfinder: BERGMANN, Kerstin, 8510 Stainz (AT); LECHNER, Doris, 8010 Graz (AT); WACHTER, Christof, 8502 Lannach (AT); WAGNER, Harald, 8055 Graz (AT); WINKLER, Heimo, 8045 Graz (AT)
(74) Vertreter: Sonn & Partner Patentanwälte

(57) **Zusammenfassung**

Die vorliegende Erfindung stellt ein Arzneimittel zur Verfügung, welches ein pharmazeutisch verträgliches Eisen(II)-Salz, Folsäure, Ascorbinsäure, und zumindest einen Hilfsstoff umfasst. Das Arzneimittel ist dadurch gekennzeichnet, dass das Eisen(II)-Salz in retardierter Form und die Ascorbinsäure in unretardierter Form vorliegen. Insbesondere ist dieses Arzneimittel zur Anwendung in der Vorbeugung gegen oder Behandlung von Eisenmangelzuständen mit Folsäuredefizit, insbesondere vier Wochen vor der Konzeption und/oder im ersten Trimenon der Schwangerschaft vorgesehen.

## Beschreibung

Das Gebiet der vorliegenden Erfindung ist das der Arzneimittel zur Behandlung von Eisenmangelzuständen mit Folsäuredefizit.

Eisen ist ein essentielles Spurenelement des Organismus. Als Coenzym der Cytochromoxidase, Katalase und Peroxidase und als Bestandteil von Hämoglobin, Myoglobin und Cytochromen ist es an zahlreichen Stoffwechselvorgängen beteiligt.

Folsäure (Vitamin B₉) muss dem Organismus in ausreichendem Maße zugeführt werden. Folsäure ist die Vorstufe des Coenzyms Tetrahydrofolsäure, welches als Carrier von C₁-Gruppen an der Biosynthese von Purinen und am Aminosäure-Stoffwechsel beteiligt ist. Damit besitzt Folsäure eine zentrale Bedeutung für den Intermediärstoffwechsel aller lebenden Zellen.

Bei dafür anfälligen Personen, z.B. bei Schwangeren, kann es zu einem Mangel an Eisen und Folsäure kommen. Dies kann unter anderem Anämie bei der Person zur Folge haben, mit Symptomen wie Müdigkeit, Depression, Blässe, Atemnot oder Herzklopfen. Im Stand der Technik sind verschiedene Folsäure/Eisen-Präparate bekannt, die gegen einen Mangel an Folsäure und Eisen eingesetzt werden können. Kombinationspräparate haben den Vorteil, dass sie die Compliance des Patienten erhöhen, weil die Einnahme gegenüber der Einnahme der jeweiligen Einzelpräparate weniger aufwändig ist.

Beispielsweise enthält eine Tablette Ferro-Folsan^{®} (Pharmazeutischer Unternehmer: Desma GmbH, Mainz-Kastel, Deutschland) laut Gebrauchsinformation 100 mg Eisen(II)-sulfat und 850 µg Folsäure. Ferro-Folsan^{®} ist laut Gebrauchsinformation zur Behandlung von nachgewiesenen, kombinierten Eisen- und Folsäuremangelzuständen in der Schwangerschaft und Stillzeit, die ernährungsmäßig nicht behoben werden können, vorgesehen.

Häufige Nebenwirkungen von Präparaten zur Behandlung von Eisen- und Folsäuremangelzuständen im Stand der Technik sind Erkrankungen des Gastrointestinaltraktes, wie Magen-Darm-Störungen, Übelkeit, Erbrechen, Magendrücken, Durchfall oder Verstopfung. Bei der Einnahme eines Eisen/Folsäure-Präparats können im Verdauungstrakt nämlich hohe lokale Eisenkonzentrationen entstehen, welche mitunter die Schleimhaut im Verdauungstrakt reizen. Außerdem kommt es bei Einnahme von herkömmlichen Präparaten oft zu unzureichender Resorption der Wirkstoffe.

Eine Aufgabe der vorliegenden Erfindung ist es daher, ein Arzneimittel zur Behandlung von Eisenmangelzuständen mit Folsäuredefizit zur Verfügung zu stellen, welches bei Einnahme möglichst geringe Nebenwirkungen verursacht bzw. eine möglichst effektive Resorption der darin beinhalteten Folsäure bzw. des Eisen(II)-Salzes im Körper des Patienten bewirkt.

Die vorliegende Erfindung stellt ein Arzneimittel zur Verfügung, welches ein pharmazeutisch verträgliches Eisen(II)-Salz, Folsäure, Ascorbinsäure, und zumindest einen Hilfsstoff umfasst. Das Arzneimittel ist dadurch gekennzeichnet, dass das Eisen(II)-Salz in retardierter Form und die Ascorbinsäure in unretardierter Form vorliegen.

Dieses Arzneimittel ist insbesondere zur Behandlung von Eisenmangelzuständen mit Folsäuredefizit hervorragend geeignet. Durch die Retardierung des Eisen(II)-Salzes werden bei der Einnahme hohe lokale Eisenkonzentrationen im Verdauungstrakt verringert. Zusätzlich bewirkt die Ascorbinsäure, wie sie erfindungsgemäß vorliegt, eine Verbesserung der Resorption des zweiwertigen Eisens.

Darüber hinaus unterstützt die unretardiert vorliegende Ascorbinsäure die Retardierung des Eisen(II)-Salzes, wie auch die Experimente von Beispiel 4 zeigen. Es hat sich insbesondere herausgestellt, dass dieser Effekt in der für Nebenwirkungen wie Übelkeit besonders wichtigen Anfangszeit der Freisetzung (d.h. in den ersten Stunden) in Relation zur Menge des bereits freigesetzten Eisen(II)-Salzes stärker ist als in späteren Phasen der Freisetzung (vgl. Beispiel 4).

Der Stand der Technik hat insbesondere diese zusätzliche technische Wirkung der Ascorbinsäure in Eisen(II)-Folsäure-Formulierungen nicht erkannt und schlägt deshalb die Retardierung der Ascorbinsäure vor bzw. lässt offen, in welcher Form die Ascorbinsäure vorliegen soll:
Die US 2006/134227 A1 bezieht sich auf eisenhältige Zusammensetzungen. Die Ausführungsbeispiele 2 und 9 offenbaren Zusammensetzungen mit Eisen(II)-Salz (Eisenfumarat), Ascorbinsäure und Folsäure. Das in diesen Zusammensetzungen ebenfalls als Inhaltsstoff genannte Ferrochel (das Chelat Eisen-Bisglycinat; vgl. auch Absatz [0019] des Dokuments) ist jedoch keine retardierte Form von Eisen (dies wird im Dokument auch nicht behauptet). Daher ist die vorliegende Erfindung allein schon aus diesem Grund nicht vorweggenommen. Ferner offenbart Ausführungsbeispiel 16 die Herstellung von Polysaccharid-Eisen-Komplex (in Form von Kügelchen, die mit Aquacoat^{®} CPD besprüht wurden) (Absatz [0056]). Zunächst ist Polysaccharid-Eisen-Komplex kein Eisen(II)-Salz. Außerdem wird dieses Ausführungsbeispiel isoliert, d.h. nicht im Zusammenhang mit weiteren Inhaltsstoffen wie Folsäure und Ascorbinsäure (oder gar unretardierter Ascorbinsäure) offenbart. Absatz [0030] des Dokuments, der sich auf Ausführungsbeispiel 16 bezieht, lässt völlig offen, ob einzelne Komponenten der Zusammensetzung nun dieselbe oder unterschiedliche (oder gar keine) Beschichtungen erhalten sollen. Somit ist evident, dass das Dokument die vorliegende Erfindung weder vorwegnimmt noch nahelegt.

In der WO 01/12163 A1 wird ein Nahrungsergänzungsmittel mit einer sich rasch auflösenden Eisenverbindung und einer sich langsam auflösenden Eisenverbindung offenbart. Die Beispiele 2-5 beschreiben kaubare bzw. nicht-kaubare Tabletten mit einem Eisen(II)-Salz, Folsäure und Ascorbinsäure. In der allgemeinen Offenbarung wird Ascorbinsäure (anders als die Vitamine A, D3, B12 und K) zusammen mit Eisen und Folsäure gegebenenfalls in der retardierten Komponente des Nahrungsergänzungsmittels verortet (S. 24, Z. 9-14). Der Ascorbinsäure wird im Dokument also eine Sonderstellung zugedacht, während Vitamine wie A, D3, B12 und K retardiert sein können. Eine eindeutige Offenbarung, gemäß derer die Ascorbinsäure in unretardierter Form vorgesehen wäre, ist im Dokument nicht enthalten. Zusammenfassend wird die vorliegende Erfindung von diesem Dokument weder vorweggenommen noch nahegelegt.

Das Dokument "Product Identification Section" (in: "Pharmaceutical Suspensions in Pharmaceutical Dosage Forms", 1989, Seiten 401-438), zeigt auf S. 403 die Filmtabletten (Filmtab^{®}) Fero-Folic-500^{®} und Iberet-Folic-500^{®} von Abott, die retardiertes Eisen mit Vitamin C und Folsäure beinhalten. Aus dem Dokument ergibt sich allerdings nicht, ob Vitamin C retardiert oder unretardiert vorliegt. Daher kann das Dokument nicht neuheitsschädlich sein. Es führt auch nicht zur vorliegenden Erfindung hin.

Darüber hinaus zeigte sich beispielsweise bei experimenteller Überprüfung des ersten in jenem Dokument beschriebenen Produkts, Fero-Folic-500^{®}, dass darin Vitamin C, anders als bei der vorliegenden Erfindung, retardiert vorliegt (vgl. Beispiel 5).

Zuletzt bezieht sich die DE 10 2005 011029 A1 (bzw. deren internationale Nachanmeldung, veröffentlicht unter WO 2006/094782 A2) auf eine Zusammensetzung für die perorale Applikation mit gesteuerter Freisetzung von Wirkstoffen, insbesondere aus der Gruppe von Vitaminen, Mineralstoffen und Spurenelementen. Ascorbinsäure ist im selben, retardierten Freisetzungssystem wie das Eisen(II)-Salz vorgesehen. Das Dokument nimmt die vorliegende Erfindung nicht vorweg noch führt es zu ihr hin.

Es hat sich des Weiteren gezeigt, dass Eisen (II)-Salz besonders gut nach der Einnahme resorbiert wird, wenn dieser Prozess nicht durch das Vorhandensein weiterer Vitamine, Mineralstoffe und/oder Spurenelemente im Arzneimittel gestört wird. Somit enthält das erfindungsgemäße Arzneimittel keine weiteren Stoffe, ausgewählt aus Vitaminen (z.B. Vitamine D, E, oder K), Mineralstoffen (z.B. zweiwertigen Metallsalzen wie Ca(II)-, Cu(II)-, Mn(II)- und Zn(II)-Salze) und/oder Spurenelementen (z.B. Cu, I, Mn, Se, Mo, Zn bzw. Verbindungen oder Salze davon) als pharmazeutische Wirkstoffe (für den Fachmann ist evident, dass sich die Gruppen der Mineralstoffe bzw. Spurenelemente überschneiden). Anders ausgedrückt enthält das erfindungsgemäße Arzneimittel in einer bevorzugten Ausführungsform als pharmazeutische Wirkstoffe im Wesentlichen nur Folsäure, ein pharmazeutisch verträgliches Eisen(II)-Salz, insbesondere Eisen(II)-Fumarat, und Ascorbinsäure.

Zur Behandlung von Eisenmangelzuständen mit FolsäureDefizit, insbesondere zur Einnahme einmal täglich, sind folgende Dosen an pharmazeutisch verträglichen Eisen(II)-Salz, Folsäure bzw. Ascorbinsäure im erfindungsgemäßen Arzneimittel besonders geeignet und daher erfindungsgemäß unabhängig voneinander oder in Kombination zu bevorzugen:
Bevorzugte Dosis (d.h. Gesamtdosis) von pharmazeutisch verträglichem Eisen(II)-Salz im erfindungsgemäßen Arzneimittel ist eine Dosis von pharmazeutisch verträglichem Eisen(II)-Salz, insbesondere Eisen(II)-Fumarat, entsprechend 10 mg - 130 mg, bevorzugt 30 mg - 110 mg, mehr bevorzugt 40 mg - 100 mg, noch mehr bevorzugt 50 mg - 90 mg, insbesondere 60 mg - 80 mg, oder gar 60 mg - 70 mg Fe²⁺.

Bevorzugte Dosis (d.h. Gesamtdosis) von Folsäure im erfindungsgemäßen Arzneimittel ist eine Dosis von 100 µg - 1500 µg, bevorzugt 300 µg - 1300 µg, mehr bevorzugt 500 µg - 1100 µg, noch mehr bevorzugt 600 µg - 1000 µg, insbesondere 700 µg - 900 µg, oder gar 750 µg - 850 µg Folsäure. Zweckmäßigerweise liegt die Folsäure in nicht-retardierter Form im erfindungsgemäßen Arzneimittel vor.

Bevorzugte Dosis (d.h. Gesamtdosis) von Ascorbinsäure im erfindungsgemäßen Arzneimittel ist eine Dosis von 10 mg - 190 mg, bevorzugt 30 mg - 170 mg, mehr bevorzugt 50 mg - 150 mg, noch mehr bevorzugt 70 mg - 130 mg, insbesondere 80 mg - 120 mg, oder gar 90 mg - 110 mg Ascorbinsäure.

Insbesondere weist das erfindungsgemäße Arzneimittel die jeweilige Dosis von pharmazeutisch verträglichem Eisen(II)-Salz, Folsäure bzw. Ascorbinsäure gemäß aller der drei vorigen Absätze auf.

Die gleichzeitige Einnahme von Ascorbinsäure verstärkt wie gesagt auch die enterale Resorption von zweiwertigem Eisen. In herkömmlichen Präparaten wird dafür so viel Ascorbinsäure vorgesehen, dass Fe²⁺ zu Ascorbinsäure in einem Massenverhältnis von ca. 1:3 bis 1:4 im Präparat vorliegt. Es hat sich jedoch unerwartet herausgestellt, dass wesentlich weniger Ascorbinsäure für eine hinreichende Resorptionsunterstützung (z.B. das Erreichen von 80%, 85% oder gar 90% der Eisenresorption, welche beim genannten Massenverhältnis von 1:4 erreicht wird) erforderlich ist. Eine Minderung der Ascorbinsäuremenge im Arzneimittel verringert das Risiko für eine Nebenwirkung bzw. die Kosten für die Herstellung. Vorteilhafterweise liegt deshalb das Massenverhältnis von Fe²⁺ zu Ascorbinsäure im erfindungsgemäßen Arzneimittel bei 1:1 bis 1:2, bevorzugt bei 1:1,1 bis 1:1,9 , mehr bevorzugt bei 1:1,2 bis 1:1,8, noch mehr bevorzugt bei 1:1,3 bis 1:1,7 , insbesondere bei 1:1,4 bis 1,6 oder gar bei im Wesentlichen 1:1,5. Beispielsweise kann die Eisenresorption zwei Wochen nach der Einnahme eines ⁵⁵Fe bzw. ⁵⁹Fe-markierten Arzneimittels in einem Ganzkörperzähler oder an einer Blutprobe mittels eines Szintillationszählers ermittelt werden.

Wie ein Wirkstoff in retardierter Form formuliert werden kann (d.h. mit verzögerter Wirkstofffreisetzung formuliert werden kann), ist im Stand der Technik bekannt, beispielsweise aus der WO 2010/028794 A1 oder der WO 2010/105824 A1. Bevorzugt wird im erfindungsgemäßen Arzneimittel ein Polymer als (Eisen-)retardierender Hilfsstoff eingesetzt, welches Polymer ausgewählt ist aus den neutralen Homo- und Co-polymeren aus (Meth)acrylsäureestern, kationischen Homo- und Co-polymeren aus (Meth)acrylsäureestern mit quartären Ammoniumgruppen, Polyvinylacetat, Celluloseacetat, Cellulosepropionat, Celluloseacetatbutyrat, Ethylcellulose, bevorzugt aus neutralen Homo- und Co-polymeren aus (Meth)acrylsäureestern, insbesondere einem Co-Polymer aus Ethylacrylat und Methylmethacrylat. Es ist vorteilhaft, wenn das Arzneimittel bzw. die Eisen(II)-hältige Komponente 50% des Eisen(II)-Gehalts erst nach mehr als 10 Minuten freigesetzt hat, bevorzugt erst nach mehr als 30 Minuten, mehr bevorzugt erst nach mehr als 50 Minuten, noch mehr bevorzugt erst nach mehr als 70 Minuten, insbesondere erst nach mehr als 80 Minuten oder gar erst nach mehr als 90 Minuten; wobei es gegebenenfalls von Vorteil ist, wenn der Eisen(II)-Gehalt nach weniger als 36 Stunden im Wesentlichen vollständig freigesetzt worden ist, bevorzugt nach weniger als 24 Stunden, mehr bevorzugt nach weniger als 18 Stunden, noch mehr bevorzugt nach weniger als 14 Stunden, insbesondere nach weniger als 12 oder gar nach weniger als 10 Stunden. Bevorzugt wird die Freisetzung von Eisen(II) nach dem in Beispiel 3 offenbarten Verfahren gemessen, wobei natürlich unter anderem die Zeitpunkte der Probenentnahmen auf dem Fachmann zweckmäßig erscheinende Weise variiert werden können.

In einer bevorzugten Ausführungsform des erfindungsgemäßen Arzneimittels ist das Eisen(II)-Salz in eine nicht-quellbare Matrix eingebettet (d.h. die Matrix schwillt in wässriger Lösung nicht auf). Die Matrix, in die das Eisen(II)-Salz eingebettet ist, hat vorzugsweise eine Quellungszahl, die unter 1,5, bevorzugt unter 1,4, mehr bevorzugt unter 1,3, noch mehr bevorzugt unter 1,2, insbesondere unter 1,1 oder gar unter 1,05 liegt, z.B. bei etwa 1. Die Quellungszahl gibt das Volumen in Millilitern an, das 1 g Stoff einschließlich des gegebenenfalls anhaftenden Schleims nach Quellen in einer wässrigen Lösung nach 4 Stunden einnimmt. Die Quellungszahl wird nach Ph. Eur. 8. Ausgabe, Kapitel 2.8.4 bestimmt.

Im Stand der Technik ist bekannt, dass Vitamine wie Folsäure (und gegebenenfalls auch andere Wirkstoffe) instabil sind und sich daher insbesondere bei längerer Lagerung eines Präparats mit Vitaminen die Dosis der Vitamine vermindern kann. Daher werden Vitamine oft im Überschuss ("Overage"), d.h. über der eigentlich empfohlenen Dosis, zugegeben.

Dies kann erhöhte Kosten für den Hersteller zur Folge haben. Des Weiteren steht zu erwarten, dass die Akzeptanz der jeweils zuständigen Arzneimittelzulassungsbehörde für solche Dosierungsüberschüsse schwinden wird.

Somit besteht in einem weiteren Aspekt die Aufgabe darin, das erfindungsgemäße Arzneimittel, insbesondere die darin enthaltene Folsäure, zu stabilisieren.

Daher umfasst das erfindungsgemäße Arzneimittel in einer bevorzugten Ausführungsform zumindest zwei separat verpresste Komponenten, wobei die erste Komponente Folsäure und die zweite Komponente pharmazeutisch verträgliches Eisen(II)-Salz beinhaltet. Im Folgenden werden die genannte erste separat verpresste Komponente bzw. die genannte zweite separat verpresste Komponente auch als die "erste, Folsäure-hältige Komponente" bzw. die "zweite, Eisen(II)-hältige Komponente" bezeichnet, oder auch einfach nur als "die erste Komponente" bzw. "die zweite Komponente".

Es hat sich im Zuge der Erfindung überraschend ergeben, dass die Folsäure im erfindungsgemäßen Arzneimittel stabilisiert wird, wenn die Möglichkeit einer Wechselwirkung zwischen dem Eisen(II)-Salz und der Folsäure eingeschränkt wird (vgl. Beispiel 2). Des Weiteren hat sich überraschend herausgestellt, dass die Ascorbinsäure im erfindungsgemäßen Arzneimittel zur Stabilisierung der Folsäure beiträgt (vgl. ebenfalls Beispiel 2). Eine Lösung, die Folsäure zu stabilisieren, ist deshalb die erwähnte separate Verpressung in zwei Komponenten, denn eine Verpressung verringert die (für eine Wechselwirkung anfällige) Oberfläche der ersten, Folsäure-hältigen bzw. der zweiten, Eisen(II)-hältigen Komponente im Verhältnis zu deren jeweiliger, für das Erreichen der jeweiligen Dosis nötigen Masse.

Für den Fachmann versteht sich, dass jede der hierin genannten separat verpressten Komponenten, jeweils unabhängig voneinander, in mehrfacher Ausführung (z.B. als mehrere Minitabletten oder als eine Vielzahl an Pellets) im erfindungsgemäßen Arzneimittel vorhanden sein kann.

Im Gegensatz zur Lehre der vorliegenden Erfindung enthalten Folsäure/Eisen-Präparate im Stand der Technik oftmals sowohl Folsäure als auch ein Eisen(II)-Salz als Granulat oder Granulatgemisch in derselben Kapsel (also mit jeweils großen, für Wechselwirkungen anfälligen Oberflächen der Folsäure- bzw. Eisen(II)-Partikel) oder sogar verpresst in derselben Komponente, wie z.B. in der WO 2006/094782 A2 offenbart. Jenes Dokument bezieht sich auf eine Zusammensetzung für die perorale Applikation mit gesteuerter Freisetzung von Wirkstoffen, insbesondere aus der Gruppe von Vitaminen, Mineralstoffen und Spurenelementen (S. 1, Zeilen 1-11 des Dokuments). Gemäß einer besonderen Ausgestaltung kann jene Zusammensetzung drei Freisetzungssysteme enthalten (S. 10, Zeilen 13-29 des Dokuments); Folsäure und Eisensalze, insbesondere Eisen(II)-Fumarat, werden als im selben Freisetzungssystem, nämlich "Freisetzungssystem B", befindlich offenbart (S. 10, Zeilen 21-25 des Dokuments). Ferner wird offenbart, dass jedes Freisetzungssystem für sich granuliert wird und die resultierenden Granulate anschließend homogen miteinander vermischt werden und zu Minitabletten verpresst werden (S. 16, Zeile 30-35). Zusammenfassend offenbart die WO 2006/094782 A2 also die gemeinsame Granulierung und Verpressung von Folsäure und Eisen(II)-Salz - im Gegensatz zur Lehre der vorliegenden Erfindung.

Eine Tablette wird durch die Verpressung eines Pulvers bzw. Granulats (bzw. Gemischen davon) hergestellt. Das Granulieren eines Pulvers (bzw. einer Pulvermischung), wie es in der Arzneimittelherstellung zur Verbesserung der Fließfähigkeit durchgeführt wird, wird nicht als Verpressung bezeichnet. Das Verpressen von Pulver zu einer Tablette, ohne dass zuvor eine Granulierung durchgeführt wurde, wird als Direktverpressung bezeichnet. Die "separate Verpressung" wird im Sinne der Erfindung gemäß einer der folgenden beiden Varianten umgesetzt:
Variante I: Eine erste Komponente, z.B. Granulat A, wird verpresst (z.B. zu einer Minitablette) und unabhängig davon (d.h. in einem anderen Arbeitsschritt oder gar in einer anderen Vorrichtung für die Verpressung) wird eine zweite Komponente, z.B. Granulat B, verpresst (z.B. zu einer Minitablette). Durch die jeweilige Verpressung entsteht eine erste separat verpresste Komponente und eine zweite separat verpresste Komponente, die dann zu einem Arzneimittel (also einer einzigen Verabreichungsform) kombiniert werden. Als Beispiel sei das Zusammenfügen von jeweils einer Minitablette (der ersten separat verpressten Komponente bzw. der zweiten separat verpressten Komponente) in eine Kapsel genannt.
Variante II: Eine erste Komponente, z.B. Pellets A, wird gemeinsam mit einer zweite Komponente, die von einer Beschichtung umgeben ist, z.B. mit Film überzogene Pellets B, verpresst. Hierbei ist allerdings zwingend vorausgesetzt, dass die Beschichtung auch nach der Verpressung im Wesentlichen erhalten bleibt (z.B. mindestens 80% oder mindestens 90% der Beschichtungsfläche), so dass sich im Wesentlichen keine Grenzfläche zwischen der ersten Komponente und der zweiten Komponente bildet. Durch die Verpressung entsteht eine erste separat verpresste Komponente und eine zweite separat verpresste Komponente, die bereits durch die Verpressung zu einem Arzneimittel (also einer einzigen Verabreichungsform) kombiniert werden. Als Beispiel sei das Verpressen von mit Film überzogenen Pellets (der ersten beschichteten Komponente und der zweiten beschichteten Komponente) zu einer Tablette genannt.

Variante I ist erfindungsgemäß gegenüber Variante II zu bevorzugen, weil eine Herstellung nach Variante I weniger fehleranfällig ist, denn beispielsweise ist keine (durch Verpressung gefährdete) Beschichtung einer Komponente nötig, um die Stabilisierung zu erreichen.

Wie erwähnt soll die Oberfläche der separat verpressten Komponenten im Verhältnis zur Masse der separat verpressten Komponenten verringert werden - wobei dieser Anforderung natürlich im Lichte anderer Anforderungen an z.B. die galenischen und pharmakologischen Eigenschaften des Arzneimittels nur in für den Fachmann sinnvoll erscheinenden Maßen entsprochen werden kann. Daher haben die erste separat verpresste Komponente bzw. die zweite separat verpresste Komponente des erfindungsgemäßen Arzneimittels, unabhängig voneinander, vorzugsweise ein Oberflächezu-Masse-Verhältnis von weniger als 4 mm²/mg, bevorzugt weniger als 3 mm²/mg, mehr bevorzugt weniger als 2,5 mm²/mg, noch mehr bevorzugt weniger als 2 mm²/mg, insbesondere weniger als 1,75 mm²/mg. Wie erwähnt, kann eine separat verpresste Komponente in mehrfacher Ausführung im Arzneimittel vorhanden sein (z.B. in Form von mehreren der gleichen Minitablette). Allerdings ist es von Vorteil (im Sinne der Oberflächenverminderung), wenn jede der Ausführungen der ersten und/oder der zweiten separat verpressten Komponente des erfindungsgemäßen Arzneimittels eine gewisse Mindestmasse aufweist, bevorzugt mehr als 1 mg, mehr bevorzugt mehr als 5 mg, noch mehr bevorzugt mehr als 10 mg, insbesondere mehr als 20 mg oder gar mehr als 30 mg.

Vorteilhafterweise liegt im erfindungsgemäßen Arzneimittel Ascorbinsäure in einer dritten separat verpressten Komponente vor, was weiter zur Stabilisierung beiträgt.

In einer weiteren bevorzugten Ausführungsform der vorliegenden Erfindung sind die erste, Folsäure-hältige Komponente und die zweite, Eisen(II)-hältige Komponente durch einen weiteren Inhaltsstoff räumlich voneinander getrennt. So kann beispielsweise eine (oder beide) der genannten Komponenten, vorzugsweise die zweite, Eisen(II)-hältige Komponente, mit dem genannten, weiteren Inhaltsstoff (z.B. einem Polymer, wie Polyvinylacetat) gegebenenfalls als Film beschichtet sein. Auch dies trägt zur Stabilisierung, insbesondere der Folsäure, bei. Vorzugsweise ist als weiterer Inhaltsstoff, insbesondere zur Beschichtung, ein Stoff vorgesehen, ausgewählt aus Hydroxypropyl-methylcellulose, Carboxymethylcellulose, Hydroxyethylcellulose, Ethylhydroxyethylcellulose, Hydroxypropylcellulose, Celluloseacetylphathalat, Hydroxypropylmethylcellulosephthalat, Celluloseacetat, Cellulosepropionat, Celluloseacetatbutyrat, Polymere der Acrylsäure und deren Salze bzw. Polyacrylate bzw. Polymethacrylate bzw. Co-Polymere davon (z.B. Eudragit^{®} E, Eudragit^{®} R, Eudragit^{®} S, Eudragit^{®} NE, Eudragit^{®} RS, Eudragit^{®} RL), Polymethacrylate, Polyvinylpyrrolidon, Polyvinylacetat-phthalat, natürliche Arten von Gummi wie Carrageenan, Zein und Schellack; bzw. Kombinationen davon.

Weiterhin ist insbesondere Ascorbinsäure dieser "weitere Inhaltsstoff" und trägt zur zumindest durch die räumliche Trennung der ersten Komponente von der zweiten Komponente zur erfindungsgemäßen Stabilisierung bei.

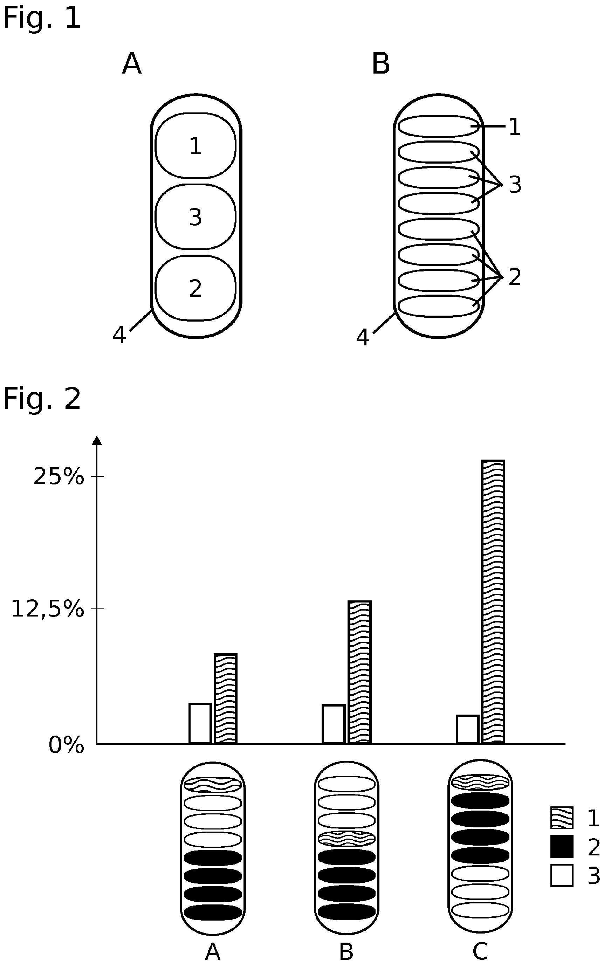

Als besonders geeignet hat sich eine Ausführungsform des erfindungsgemäßen Arzneimittels erwiesen, bei der Ascorbinsäure in der dritten separat verpressten Komponente vorliegt, und die erste Komponente (welche Folsäure beinhaltet) und die zweite Komponente (welche pharmazeutisch verträgliches Eisen(II)-Salz beinhaltet) durch die dritte Komponente räumlich voneinander getrennt sind (vgl. auch Fig. 1). Dies verringert allein schon durch die räumliche Trennung die Möglichkeit der Wechselwirkung zwischen Folsäure und Eisen(II)-Salz. Zusätzlich kann sich die dritte, Ascorbinsäure-hältige Komponente dadurch in räumlicher Nähe zu den anderen beiden genannten Komponenten - der ersten und der zweiten Komponente - befinden, und auf diese stabilisierend einwirken.

Es ist besonders zweckmäßig für die Stabilisierung insbesondere der Folsäure, wenn das erfindungsgemäße Arzneimittel als Kapsel vorliegt. Darin liegen die erste und die zweite Komponente, gegebenenfalls auch die dritte, Ascorbinsäure-hältige Komponente, bevorzugt als Minitablette(n) vor. Die besagten Komponenten liegen gegebenenfalls jeweils in mehrfacher Ausführung vor. Bevorzugt umfasst die erste, Folsäure-hältige Komponente als Hilfsstoffe ein Füllmittel wie Lactose, ein Zerfallsmittel wie mikrokristalline Cellulose, ein Fließmittel wie kolloidale Kieselsäure und/oder ein Schmiermittel wie Magnesiumstearat. Bevorzugt umfasst die zweite, Eisen (II) -hältige Komponente als Hilfsstoffe ein Füllmittel wie Lactose, ein Retardierungsmittel wie ein Copolymer von Ethylacrylat und Methylmethacrylate, ein Fließmittel wie kolloidale Kieselsäure und/oder ein Schmiermittel wie Magnesiumstearat. Die Hülle der Kapsel kann beispielsweise im Wesentlichen aus Hartgelatine bestehen. Vorzugsweise hat eine Minitablette erfindungsgemäß eine Masse zwischen 10 mg bis 100 mg, bevorzugt zwischen 20 mg bis 90 mg, mehr bevorzugt zwischen 30 mg bis 80 mg, insbesondere zwischen 40 mg und 70 mg und/oder einen Durchmesser von 0,25 mm bis 7 mm, bevorzugt von 0,5 mm - 6 mm, insbesondere von 1 mm - 5 mm.

In einer ganz besonders bevorzugten Ausführungsform der vorliegenden Erfindung ist das erfindungsgemäße Arzneimittel eine Kapsel, worin die erste separat verpresste, Folsäure-hältige Komponente und die zweite separat verpresste, Eisen(II)-hältige Komponente jeweils als eine oder mehrere Minitabletten vorliegen. In dieser Ausführungsform liegt Ascorbinsäure in einer dritten, separat verpressten Komponente als eine oder mehrere Minitablette(n) vor, diese dritte Komponente ist zwischen der ersten separat verpressten Komponente und der zweiten separat verpressten Komponente in der Kapsel gelegen. Die erste und die zweite separat verpresste Komponente werden somit in dieser Ausführungsform durch die dritte separat verpresste Komponente räumlich voneinander getrennt (vgl. auch Fig. 1).

In einer weiteren bevorzugten Ausführungsform der vorliegenden Erfindung liegt das erfindungsgemäße Arzneimittel als Tablette vor. Die erfindungsgemäße Stabilisierung wird bevorzugt dadurch erreicht, dass die erste und die zweite Komponente, gegebenenfalls auch die dritte, Ascorbinsäure-hältige Komponente, jeweils in mehrfacher Ausführung als Pellets vorliegen. Bevorzugt weist zumindest eine der genannten Komponenten, insbesondere die zweite, Eisen(II)-hältige Komponente, (gegebenenfalls in mehrfacher Ausführung) eine Beschichtung auf. Beispielsweise kann die zweite, Eisen(II)-hältige Komponente in Form von Pellets vorliegen, welche jeweils einzeln beschichtet sind (d.h. es kann auch von einer Komponente gesprochen werden, die beschichtet ist bzw. eine Beschichtung aufweist). Insbesondere liegt das erfindungsgemäße Arzneimittel in Form einer Tablette als Multiple Unit Pellet System (auch als MUPS^{®} bezeichnet) vor. Multiple Unit Pellet Systeme sind beispielsweise bekannt aus der WO 2011/107921 A2, der WO 2013/092497 A1 oder der WO 2014/016396 A1.

Im Sinne der vorliegenden Erfindung kann jedes pharmazeutisch verträgliche bzw. annehmbare Eisen(II)-Salz für das erfindungsgemäße Arzneimittel gewählt werden. Bevorzugt ist das Eisen(II)-Salz ausgewählt aus Eisen(II)-Fumarat, -sulfat, - gluconat, -lactat, -tartrat, -succinat, -glutamat, -citrat,-pyrophosphat, -carbonat, und Kombinationen davon. Insbesondere liegt das Eisen(II)-Salz als Eisen (II) -Fumarat vor, welches sich als sehr geeignet für die Zwecke der vorliegenden Erfindung erwiesen hat.

Als Hilfsstoff umfasst das erfindungsgemäße Arzneimittel (bzw. eine oder alle getrennt verpressten Komponenten im Arzneimittel) beispielsweise einen Stoff, ausgewählt aus der Gruppe der Polysaccharide, wie Cellulose (z.B. mikrokristalline Cellulose) Hydroxypropylmethylcellulose, Carboxymethylcellulose, Hydroxyethylcellulose, Ethylhydroxyethylcellulose, Hydroxypropylcellulose, Celluloseacetylphathalat, Hydroxypropylmethylcellulosephthalat, Celluloseacetat, Cellulosepropionat, Celluloseacetatbutyrat, Ethylcellulose, Guar-Mehl, Alginsäure und/oder Alginate, Pektin, Polyvinylpyrrolidon Polyvinylacetat, Polyvinylalkohol, Polyvinylchlorid, Polymere der Acrylsäure und deren Salze bzw. Polyacrylate bzw. Polymethacrylate bzw. Co-Polymere davon (z.B. Eudragit^{®} E, Eudragit^{®} R, Eudragit^{®} S, Eudragit^{®} NE, Eudragit^{®} RS, Eudragit^{®} RL), Vinylpyrrolidon-VinylacetatCopolymere, Nylon, kolloidale Kieselsäure (z.B. Aerosil^{®}), Behenate, Stearate, Polyamid, Polyacrylamid, Polyethylen, Polyalkylenglykole wie Polyethylenglykol, Co-polymere von Polyalkylenglykolen, z.B. von Polyethylenglykol und Polypropylenglykol (Pluronic^{®}, BASF), Zucker, Zuckeralkohol; und Gemischen davon. Dem Fachmann sind weitere geeignete Hilfsstoffe vertraut.

Das erfindungsgemäße Arzneimittel ist bevorzugt zur Anwendung in der Vorbeugung gegen, oder Behandlung von, Eisenmangelzuständen mit Folsäuredefizit, insbesondere vier Wochen vor der Konzeption und/oder im ersten Trimenon der Schwangerschaft.

Gerade für Schwangere (insbesondere im ersten Trimenon, weil dieser für die Neuralrohrentwicklung essentiell ist) bzw. vier Wochen vor der Konzeption (um bis zur Konzeption einen entsprechenden Spiegel an Eisen bzw. Folsäure aufzubauen) ist das erfindungsgemäße Arzneimittel besonders gut zur täglichen Einnahme geeignet. Ein Mangel an Eisen bzw. Folsäure in der Schwangerschaft kann verschiedene Komplikationen verursachen, die sowohl Mutter als auch das Kind betreffen. Neben Anämie umfassen diese Komplikationen vorzeitige Wehentätigkeit oder niedriges Geburtsgewicht, in schweren Fällen auch Fehlbildungen wie Neuralrohrdefekte oder Lippen-/Kiefer-/Gaumenspalten.

Ein Serum-Folatspiegel bei Erwachsenen von 7-20 nmol/L wird üblicherweise als normal erachtet, bei Werten darunter liegt ein Folsäuremangel vor. Eisenmangel wird üblicherweise indirekt durch Quantifizierung von Hämoglobin, Ferritin (wird als besonders geeignet erachtet), Erythrozyten und/oder Retikulozyten im Blut ermittelt, die Normalwerte (Referenzbereiche) für Frauen liegen gemäß einer Definition bei 12-16 g/dl Hämoglobin, 23-110 µg/l Ferritin, 3,9-5,7 Mio./µl Erythrozyten und davon 3-18 Promille Retikulozyten.

Das erfindungsgemäße Arzneimittel ist bevorzugt zur peroralen Verabreichung vorgesehen, insbesondere 1x täglich.

Der Ausdruck "stabilisiert" bzw. "stabilisieren" bzw. "Stabilisierung", wie hierin gebraucht, ist im relativen Sinn zu verstehen. D.h. trotz Stabilisierung einer Substanz kann es zu einem Abbau der Substanz kommen. Die Substanz gilt auch als stabilisiert, wenn dieser Abbau gegenüber einer geeigneten Kontrolle nur verlangsamt wird.

Die vorliegende Erfindung wird durch die folgenden Beispiele und die Figuren illustriert, auf welche sie selbstverständlich nicht eingeschränkt ist.
**Figur 1****: (A)** Eine Hartgelatine-Kapsel (4) beinhaltet die erste separat verpresste, Folsäure-hältige Komponente (1) in Form einer Minitablette, die zweite separat verpresste, Eisen(II)-hältige Komponente (2) in Form einer Minitablette, und Ascorbinsäure in einer dritten separat verpressten Komponente (3) in Form einer Minitablette. Komponente (3) trennt die Komponenten (1) und (2) räumlich voneinander. **(B)** Eine Hartgelatine-Kapsel (4) beinhaltet die erste separat verpresste, Folsäure-hältige Komponente (1) in Form einer Minitablette, die zweite separat verpresste, Eisen(II)-hältige Komponente (2) in Form von vier Minitabletten, und Ascorbinsäure in einer dritten separat verpressten Komponente (3) in Form von drei Minitabletten. Komponente (3) trennt die Komponenten (1) und (2) räumlich voneinander.
**Figur 2****:** Kapseln wurden jeweils mit einer Folsäure-hältigen Minitablette (1), vier Eisen(II)-Fumarat-hältigen Minitabletten (2) und drei Ascorbinsäure-hältigen Minitabletten (3), in den Anordnungen A (Ascorbinsäure-hältige Minitabletten in der Mitte), B (Folsäure-hältige Minitablette in der Mitte) oder C (Eisen(II)-Fumarat-hältige Minitabletten in der Mitte) befüllt. Nach drei Monaten wurden die Kapseln auf den Restgehalt von Folsäure bzw. Ascorbinsäure hin untersucht. Die Anordnung der Minitabletten in der Kapsel hat einen starken Einfluss auf die Stabilität der Folsäure: in Kapseln mit Anordnung A wurde in der Folsäure-hältigen Minitablette ein Folsäuregehaltsverlust (mit Wellen schraffierte Balken) von 8% festgestellt, in Kapseln mit Anordnung B ein Verlust von 13% und in Kapseln mit Anordnung C sogar ein Verlust von 26%. Im Vergleich dazu hatte die Anordnung in der Kapsel hingegen deutlich geringeren Einfluss auf den Ascorbinsäuregehalt - der Ascorbinsäuregehaltsverlust (weiße Balken) nach drei Monaten lag für alle Anordnungen zwischen 2,5% und 3,5%.

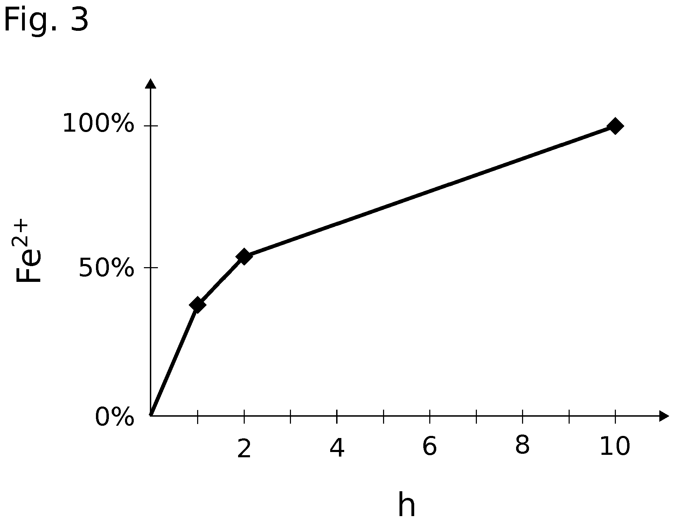

**Figur 3****:** Freisetzungsverhalten einer retardierten, gemäß Beispiel 1 hergestellten Eisen(II)-Fumarat-hältigen Minitablette in künstlichem Magensaft ohne Pepsin bei 37°C und Agitation. Nach einer Stunde waren etwa 40% des ursprünglich in der Minitablette beinhalteten Eisen(II) freigesetzt, nach zwei Stunden etwa 50% und nach 10 Stunden 100% (n=6).

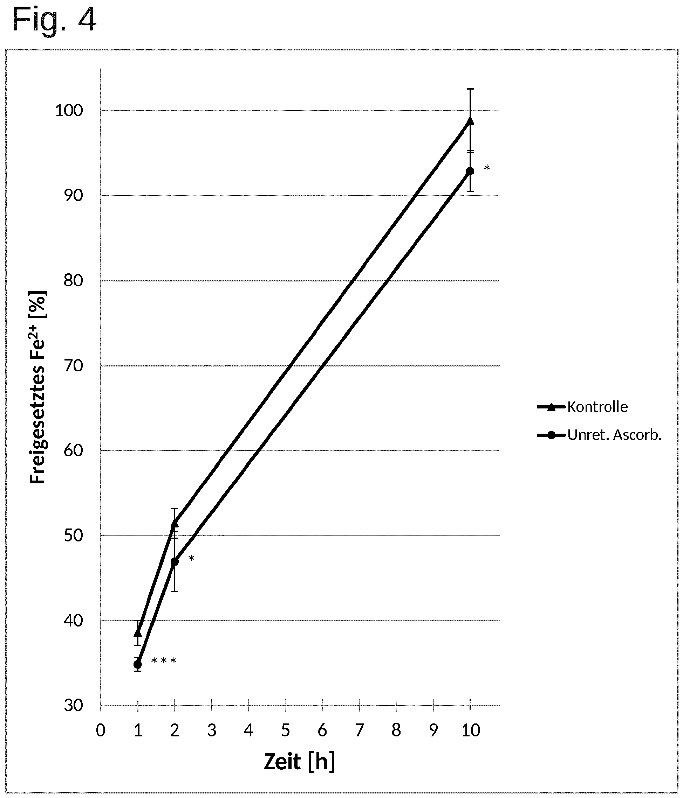

**Figur 4****:** Freisetzungsverhalten von retardierten, gemäß Beispiel 1 hergestellten Eisen(II)-Fumarat-hältigen Minitabletten in künstlichem Magensaft ohne Pepsin bei 37°C und Agitation, in Abwesenheit Kontrolle") oder Anwesenheit ("Unret. Ascorb") von nicht-retardierten, gemäß Beispiel 1 hergestellten Ascorbinsäure-Minitabletten. Wie sich herausgestellt hat, unterstützt unretardierte Ascorbinsäure die Retardierung von Eisen(II)-Salz. * p<0,05, *** p<0,0005 (t-Test, zweiseitig)

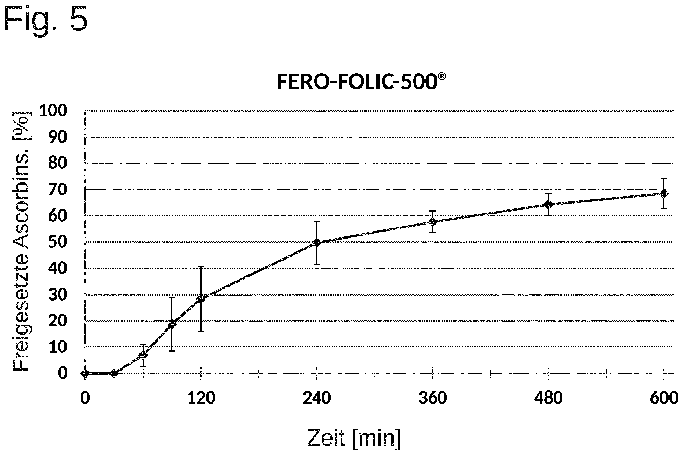

**Figur 5****:** Ascorbinsäure-Freisetzungsprofil bei Fero-Folic-500^{®}-Tabletten. Am Freisetzungsprofil wird evident, dass Ascorbinsäure in Fero-Folic-500^{®}-Tabletten, anders als bei der vorliegenden Erfindung, retardiert vorliegt. Nach einer Stunde ist weniger als 10% der Gesamtmenge an Ascorbinsäure freigesetzt worden, nach zwei Stunden erst knapp 30%, nach vier Stunden erst die Hälfte.

### Beispiel 1 - Herstellung des Arzneimittels

Ein Arzneimittel in Form einer lichtundurchlässigen Hartgelatine-Kapsel, beinhaltend eine Folsäure-hältige Minitablette (nicht-retardiert), vier Eisen(II)-Fumarat-hältige Minitabletten (retardiert) und drei Ascorbinsäure-hältige Minitabletten (nicht-retardiert), soll hergestellt werden. Die jeweiligen Gesamtdosen in einer Kapsel sollen in etwa betragen: 800 µg Folsäure, 202, 6 mg Eisen(II)-Fumarat (entsprechend 66,6 mg Fe²⁺) (zu gleichen Teilen aufgeteilt auf 4 Minitabletten) und 100 mg Ascorbinsäure (zu gleichen Teilen aufgeteilt auf 3 Minitabletten). Hierzu wurde wie folgt verfahren:
Herstellung von 5000 Stück Folsäure-Minitabletten: 4,76 g Folsäure (Gehalt 91,5%), 120,02 g Lactose, 120,23 g mikrokristalline Cellulose, 2,49 g kolloidale Kieselsäure und 2,5 g Magnesiumstearat wurden gemischt. Die so erhaltene Pulvermischung wurde zu 5000 Minitabletten zu je 50 mg direktverpresst (Stempel: 5 mm rund, Tablettendicke ca. 2 mm; Exzenterpresse Korsch EK 0, Korsch AG, Berlin).

Herstellung von 5000 Stück Eisen(II)-Fumarat-Minitabletten: 272,5 g Eisen(II)-Fumarat (Gehalt 93%) und 17,1 g Lactose-Monohydrat wurden gemischt und unter Zugabe von 77,65 g Polyacrylat Dispersion 30% (Trockenmasse: 23,3 g) granuliert. Das Granulat wurde getrocknet und gesiebt. Hernach wurden 3 g kolloidale Kieselsäure und 3 g Magnesiumstearat hinzugemischt. Das so erhaltene Granulat wurde zu 5000 Minitabletten zu je 63,8 mg direktverpresst (Stempel: 5 mm rund, Tablettendicke ca. 2 mm; Exzenterpresse Korsch EK 0, Korsch AG, Berlin).

Herstellung von 5000 Stück Ascorbinsäure-Minitabletten: 167 g Ascorbinsäure (Gehalt 99,7%), 19,56 g Lactose, 58,67 g mikrokristalline Cellulose, 2,53 g kolloidale Kieselsäure und 2,52 g Magnesiumstearat wurden gemischt. Die so erhaltene Pulvermischung wurde zu 5000 Minitabletten zu je 50 mg direktverpresst (Stempel: 5 mm rund, Tablettendicke ca. 2 mm; Exzenterpresse Korsch EK 0, Korsch AG, Berlin).

Die Minitabletten wurden im Verhältnis 1:4:3 (Folsäure-Minitablette : Eisen(II)-Fumarat-Minitabletten : Ascorbinsäure-Minitabletten) pro Kapsel mit einer Kapselfüllmaschine in Hartgelatinekapseln verfüllt. Die Minitabletten wurden mit den Ascorbinsäure-Minitabletten in der Mitte (vgl. Fig. 2, Anordnung A) angeordnet, da diese Anordnung sehr geeignet war, um ein stabilisiertes Arzneimittel zu erhalten.

### Beispiel 2 - Stabilitätsuntersuchungen

Kapseln wurden jeweils mit einer Folsäure-hältigen Minitablette (1), vier Eisen(II)-Fumarat-hältigen Minitabletten (2) und drei Ascorbinsäure-hältigen Minitabletten (3), in den Anordnungen A (Ascorbinsäure-hältige Minitabletten in der Mitte), B (Folsäure-hältige Minitablette in der Mitte) oder C (Eisen(II)-Fumarat-hältige Minitablette in der Mitte) befüllt (vgl. Fig. 2). Die jeweiligen Minitabletten wurden gemäß Beispiel 1 hergestellt.

Die Kapseln wurden für drei Monate unter den folgenden Umweltbedingungen in einem offenen Kunststoffbehälter gelagert: Temperatur 40°C, bei 75% relativer Luftfeuchtigkeit. Danach wurden die Kapseln geöffnet und die darin befindlichen Folsäure-hältigen bzw. Ascorbinsäure-hältigen Minitabletten auf den Folsäurerestgehalt bzw. auf den Ascorbinsäurerestgehalt hin untersucht:

### Folsäurerestgehalt (bzw. Folsäuregehaltsverlust)

### Lösungen zum Herstellen:

28,6 g/L Natriumcarbonat Lösung: 28,6 g Natriumcarbonat wurden in 1000 ml H₂O dest. gelöst.
Puffer: 11,16 g Kaliumdihydrogenphosphat und 5,50 g Dikaliumhydrogenphosphat wurden in 1000 ml H₂O dest. gelöst. Es wurden 4 L Puffer hergestellt.

| | |
|---|---|
| Laufmittel (LM): | 3520 ml Puffer |
| | 480 ml Methanol |

### Probelösung:

10 Folsäure-hältige Minitabletten wurden einzeln gewogen und in einen 20 ml Maßkolben überführt. 1 ml einer 28,6 g/L Na₂CO₃-Lösung wurden dazupipettiert und mit Laufmittel bis zur Marke aufgefüllt. Danach wurde die Probe für 15 min ins Ultraschallbad gestellt. Dann wurde die Probe durch einen 0,45 µm PA Spritzenfilter in HPLC-Vials filtriert. Es wurden zwei Probelösungen hergestellt.

### Standardlösung:

10,0 mg Folsäure Referenzsubstanz, genau gewogen, wurden in einen 25 ml Maßkolben überführt und in 1,25 ml einer 28,6 g/L Na₂CO₃-Lösung gelöst. Mit Laufmittel wurde bis zur Marke aufgefüllt und für 15 min ins Ultraschallbad gestellt. Danach wurde durch einen 0,45 µm PA Spritzenfilter in HPLC-Vials filtriert. Es wurden zwei Standardlösungen hergestellt.

**HPLC-Bedingungen:**

| | | |
|---|---|---|
| Säule: | PG 101 Agilent^{®} LiChrospher^{®} 100RP-8, 5 µm; | |
| | 250x4 mm | |
| LM: | LM A: | 88 VT Puffer 90% LM B: Methanol 10% |
| | | 12 VT MeOH |
| Fluss: | | 0,6 ml/min |
| Detektion: | | 280 nm |
| Injektionsvolumen: | | 5 µl |
| Auto sampler: | | 5°C |
| Run Time: | | 20 min |

### Ascorbinsäurerestgehalt (bzw. Ascorbinsäuregehaltsverlust)

**Lösungen zum Herstellen:**

| | |
|---|---|
| Phosphatpuffer: 13,6 g Kaliumdihydrogenphosphat werden in 2000 ml H₂O dest. gelöst. | |
| Laufmittel: | 1000 ml Phosphatpuffer |
| | 3000 ml Acetonitril |

### Probelösung:

10 Ascorbinsäure-hältige Minitabletten wurden in einen 100 ml Maßkolben genau gewogen, mit Laufmittel bis zur Marke aufgefüllt und 15 min ins Ultraschallbad gegeben. Danach wurde durch einen 0,45 µm PA Spritzenfilter in HPLC-Vials filtriert. Es wurden 2 Probelösungen hergestellt.

### Standardlösung:

166,5 mg Ascorbinsäure (W=100%) wurden in einen 50 ml Maßkolben eingewogen, mit Laufmittel bis zur Marke aufgefüllt und 15 min ins Ultraschallbad gestellt. Danach wurde durch einen 0,45 µm PA Spritzenfilter in HPLC-Vials filtriert. Es wurden zwei Standardlösungen hergestellt.

**HPLC-Bedingungen:**

| | | |
|---|---|---|
| Säule: | PG 100 Macherey-Nagel Nucledur^{®} 100-5 NH2-RP | |
| Fluss: | | 1,0 ml/min |
| Detektion: | | 210 nm |
| Injektionsvolumen: | | 20 µl |
| Auto sampler: | | 5°C |
| Run Time: | | 35 min |

### Resultate

Die Anordnung der Minitabletten in der Kapsel hatte einen starken Einfluss auf die Stabilität der Folsäure: In Kapseln mit den Ascorbinsäure-hältigen Minitabletten in der Mitte ("Anordnung A") wurde in der Folsäure-hältigen Minitablette ein Folsäuregehaltsverlust von 8% festgestellt, in Kapseln mit der Folsäure-hältigen Minitablette in der Mitte ("Anordnung B") ein Verlust von 13% und in Kapseln mit den Eisen(II)-Fumarat-hältigen Minitabletten ("Anordnung C") sogar ein Verlust von 26%. Im Vergleich dazu hatte die Anordnung in der Kapsel hingegen deutlich geringeren Einfluss auf den Ascorbinsäuregehalt - der Ascorbinsäuregehaltsverlust nach drei Monaten lag für alle Anordnungen zwischen 2,5% und 3,5%. Die Ergebnisse der Stabilitätsuntersuchungen sind auch in Fig. 2 dargestellt.

Daraus sind die folgenden, im Lichte des Standes der Technik überraschenden Rückschlüsse zu ziehen: Zwischen Folsäure und Eisen(II)-Salzen kommt es bei räumlicher Nähe bzw. Grenzflächenbildung zu Wechselwirkungen, welche die Folsäure destabilisieren (siehe Fig. 2, Anordnungen B und C). Gleichzeitige Nähe bzw. Grenzflächenbildung der Folsäure mit Ascorbinsäure mildert jedoch diese destabilisierende Wechselwirkung ab (siehe Fig. 2, Anordnung B), die Ascorbinsäure hat also einen zusätzlichen stabilisierenden Effekt auf die Folsäure. In dieser Vergleichsserie am stabilsten ist eine Anordnung der Minitabletten in der Kapsel gemäß Fig. 2, Anordnung A.

### Beispiel 3 - Freisetzungsverhalten der retardierten Eisen(II)-Fumarat-hältigen Minitablette

Eine gemäß Beispiel 1 hergestellte Eisen(II)-Fumarathältige Minitablette wurde in sechsfachem Ansatz in einem VanKel^{®} VK-7000 Lösungssystem (Varian, Inc.) auf ihr Freisetzungsverhalten hin untersucht. Die Bedingungen für jeden Ansatz waren: 900 ml künstlicher Magensaft ohne Pepsin nach Ph. Eur. als Medium zur Lösung der Minitablette, Rühren mit Paddel bei 100 Umdrehungen pro Minute und einer Temperatur von 37°C. Nach ein, zwei und zehn Stunden wurden Proben aus dem Medium gezogen und auf den Eisen(II)-Gehalt hin untersucht. Der Messung der Konzentration von Eisen(II) in den Proben erfolgte mittels kolorimetrischem 2,2'-Bispyridin-Nachweis.

Nach einer Stunde waren etwa 40% des ursprünglich in der Minitablette enthaltenen Eisen(II) freigesetzt, nach zwei Stunden etwa 50% und nach 10 Stunden 100%.

### Beispiel 4 - Freisetzungsverhalten der retardierten Eisen(II)-Fumarat-hältigen Minitabletten in Ab- bzw. Anwesenheit von Ascorbinsäure-Minitabletten

Das Freisetzungsverhalten von retardierten Eisen(II)-Fumarat-hältigen Minitabletten wurde mit dem Freisetzungsverhalten der gleichen Minitabletten in der Anwesenheit von Ascorbinsäure-Minitabletten verglichen. Bei der Kontrollgruppe (gemäß Beispiel 1 hergestellte Eisen(II)-Fumarat-hältige Minitabletten) wurde im Wesentlichen wie in Beispiel 3 verfahren (6-facher Ansatz). Bei einer zweiten Versuchsgruppe wurde ebenso vorgegangen, jedoch war zusätzlich zu den Eisen(II)-Minitabletten pro Ansatz im Gefäß unretardierte Ascorbinsäure in Minitablettenform (gemäß Beispiel 1 hergestellt) vorhanden.

Für die Kontrolle konnte das Ergebnis von Beispiel 3 bzw. Fig. 3 reproduziert werden: Nach einer Stunde waren etwa 40% des ursprünglich in der Minitablette beinhalteten Eisen(II) freigesetzt, nach zwei Stunden etwa 50% und nach 10 Stunden etwa 100% (vgl. Fig. 4, obere Messkurve).

In Anwesenheit der nicht-retardierten Ascorbinsäure-Minitabletten ergaben sich jedoch signifikante Unterschiede in der Freisetzung von Fe²⁺: Nach 1 Stunde war 10% weniger Fe²⁺ freigesetzt als zum selben Zeitpunkt bei der Kontrolle, nach 2 Stunden 9% weniger als bei der Kontrolle und nach 10 Stunden 6% weniger als bei der Kontrolle (vgl. Fig. 4, untere Messkurve).

Folglich unterstützt das Vorhandensein von unretardierter Ascorbinsäure (die durch die schnelle Freisetzung innerhalb kürzester Zeit die volle Aktivität erlangt) die Retardierung von Eisen(II)-Fumarat, besonders in den ersten Stunden nach Auflösungsbeginn.

### Beispiel 5 - Freisetzungsverhalten der Ascorbinsäure bei Fero-Folic-500⁰-Tabletten

Fero-Folic-500^{®}-Tabletten (Wirkstoffe: 105 mg Eisen(II) in Form von Eisen(II)-Sulfat, 500 mg Vitamin C in Form von Natriumascorbat, 350 µg Folsäure) wurden über eine internationale Apotheke bezogen (Batch-Nr. 1601T) und hinsichtlich ihres Ascorbinsäure-Freisetzungsverhaltens überprüft. Die Tabletten wurden in sechsfachem Ansatz in einem Dissolution-Tester (Erweka GmbH, Deutschland) auf ihr Freisetzungsverhalten hin untersucht. Die Bedingungen für jeden Ansatz waren: 1L Medium (750ml 0,1M HCl + 250 ml 0,2M Na₃PO₄) zur Lösung der Tablette, und Rühren bei 100 Umdrehungen pro Minute. In verschiedenen Zeitabständen wurden Proben gezogen und mit HPLC analysiert. Die Detektion zur Ermittlung der Ascorbinsäurekonzentration erfolgte bei einer Wellenlänge von 220 nm.

Am Freisetzungsprofil zeigt sich klar, dass Ascorbinsäure in Fero-Folic-500^{®}-Tabletten, anders als bei der vorliegenden Erfindung, retardiert vorliegt. Nach einer Stunde war weniger als 10% der Gesamtmenge an Ascorbinsäure freigesetzt, nach zwei Stunden erst knapp 30%, nach vier Stunden erst die Hälfte (vgl. Fig.5).

## Patentansprüche

1. Arzneimittel, umfassend ein pharmazeutisch verträgliches Eisen(II)-Salz, Folsäure, Ascorbinsäure, und zumindest einen Hilfsstoff,
**dadurch gekennzeichnet, dass** das Eisen(II)-Salz in retardierter Form und die Ascorbinsäure in unretardierter Form vorliegen.

2. Arzneimittel gemäß Anspruch 1, **dadurch gekennzeichnet, dass** es keine weiteren Vitamine, Mineralstoffe und/oder Spurenelemente als pharmazeutische Wirkstoffe enthält.

3. Arzneimittel gemäß einem der vorhergehenden Ansprüche, **dadurch gekennzeichnet, dass** die darin vorhandene Dosis des pharmazeutisch verträglichen Eisen(II)-Salzes 10 mg - 130 mg, bevorzugt 30 mg - 110 mg, mehr bevorzugt 40 mg - 100 mg, noch mehr bevorzugt 50 mg - 90 mg, insbesondere 60 mg - 80 mg, oder gar 60 mg - 70 mg Fe²⁺ entspricht.

4. Arzneimittel gemäß einem der vorhergehenden Ansprüche, **dadurch gekennzeichnet, dass** die darin vorhandene Dosis der Folsäure 100 µg - 1500 µg, bevorzugt 300 µg - 1300 µg, mehr bevorzugt 500 µg - 1100 µg, noch mehr bevorzugt 600 µg - 1000 µg, insbesondere 700 µg - 900 µg, oder gar 750 µg - 850 µg beträgt.

5. Arzneimittel gemäß einem der vorhergehenden Ansprüche, **dadurch gekennzeichnet, dass** die darin vorhandene Dosis der Ascorbinsäure 10 mg - 190 mg, bevorzugt 30 mg - 170 mg, mehr bevorzugt 50 mg - 150 mg, noch mehr bevorzugt 70 mg - 130 mg, insbesondere 80 mg - 120 mg, oder gar 90 mg - 110 mg beträgt.

6. Arzneimittel gemäß einem der vorhergehenden Ansprüche, **dadurch gekennzeichnet, dass** das Massenverhältnis von Fe²⁺ zu Ascorbinsäure im Arzneimittel bei 1:1 bis 1:2 liegt, bevorzugt bei 1:1,1 bis 1:1,9 , mehr bevorzugt bei 1:1,2 bis 1:1,8, noch mehr bevorzugt bei 1:1,3 bis 1:1,7 , insbesondere bei 1:1,4 bis 1,6 oder gar bei im Wesentlichen 1:1,5.

7. Arzneimittel gemäß einem der vorhergehenden Ansprüche, **dadurch gekennzeichnet, dass** ferner die Folsäure in nicht-retardierter Form vorliegt.

8. Arzneimittel gemäß einem der vorhergehenden Ansprüche, **dadurch gekennzeichnet, dass** ein das Eisen(II)-Salz retardierender Hilfsstoff ein Polymer ist, ausgewählt aus den neutralen Homo- und Co-polymeren aus (Meth)acrylsäureestern, kationischen Homo- und Co-polymeren aus (Meth)acrylsäureestern mit quartären Ammoniumgruppen, Polyvinylacetat, Celluloseacetat, Cellulosepropionat, Celluloseacetatbutyrat, Ethylcellulose, bevorzugt aus neutralen Homo- und Co-polymeren aus (Meth)acrylsäureestern, insbesondere einem Co-Polymer aus Ethylacrylat und Methylmethacrylat.

9. Arzneimittel gemäß einem der vorhergehenden Ansprüche, **dadurch gekennzeichnet, dass** das Eisen(II)-Salz in eine nicht-quellbare Matrix eingebettet ist.

10. Arzneimittel gemäß einem der vorhergehenden Ansprüche, **dadurch gekennzeichnet, dass** es zumindest zwei separat verpresste Komponenten umfasst, wobei die erste Komponente Folsäure und die zweite Komponente pharmazeutisch verträgliches Eisen(II)-Salz beinhaltet.

11. Arzneimittel gemäß Anspruch 10, **dadurch gekennzeichnet, dass** die erste Komponente und die zweite Komponente durch einen weiteren Inhaltsstoff räumlich voneinander getrennt sind.

12. Arzneimittel gemäß Anspruch 11, **dadurch gekennzeichnet, dass** Ascorbinsäure in einer dritten separat verpressten Komponente vorliegt, und die erste Komponente und die zweite Komponente durch die dritte Komponente räumlich voneinander getrennt sind.

13. Arzneimittel gemäß Anspruch 10 oder 11, **dadurch gekennzeichnet, dass** es als Kapsel vorliegt.

14. Arzneimittel gemäß Anspruch 13, **dadurch gekennzeichnet, dass** besagte Komponenten als Minitabletten vorliegen.

15. Arzneimittel gemäß einem der vorhergehenden Ansprüche zur Anwendung in der Vorbeugung gegen oder Behandlung von Eisenmangelzuständen mit Folsäuredefizit, insbesondere vier Wochen vor der Konzeption und/oder im ersten Trimenon der Schwangerschaft.
